# EUROPEAN PATENT APPLICATION

(11) **EP 4 506 015 A1**
(43) Date of publication of application: **12.02.2025**
(21) Application number: 23190473.1
(22) Date of filing: 09.08.2023
(51) Int. Cl.: A61K 45/06, A61K 47/68, A61K 31/519, A61P 35/00

(54) **COMBINATION OF A MPS1 INHIBITOR AND AN ANTIBODY DRUG CONJUGATE**

(71) Applicant: Nerviano Medical Sciences S.r.l., 20014 Nerviano (MI) (IT)
(72) Inventor: MAHNKE, Lisa, Boston, 02130 (US); GASPARRI, Fabio, 20015 Parabiago (IT); TEXIDO ROMERO, Gemma, 20014 Nerviano (IT)
(74) Representative: Cattaneo, Elisabetta

(57) **Abstract**

The invention relates to a combination of a MPS1 inhibitor and an antibody drug conjugate. More particularly, the invention relates to a combination of N-(2,6-diethyl phenyl)-8-({4-[4-(dimethylamino)piperidin-1-yl]-2-methoxyphenyl}amino)-1-methyl-4,5-dihydro-1H- pyrazolo[4,3-h]quinazoline-3-carboxamide or one of its salts with a pharmaceutically acceptable acid or base and an antibody drug conjugate wherein the payload is a tubulin inhibitor agent. The invention also relates to the use of said combination in the treatment of cancer.

## Description

### FIELD OF THE INVENTION

The invention concerns synergic combination of a MPS1 inhibitor and an antibody drug conjugate wherein the payload is a tubulin inhibitor agent and the use of said combination in the treatment of cancer.

### STATE OF THE ART

Cancer is characterized by uncontrolled cell proliferation. As a novel bio-targeting antitumor agent, the antibody-drug conjugate (ADC) combines the high selectivity of monoclonal antibody and potent cytotoxicity of drug or payload. The drug release mechanism of ADCs consists of several key steps. The ADC first binds to the tumor-associated antigen on the target cells. The resulting antigen-ADC complex is internalized by receptor-mediated endocytosis, and subsequently transferred to late endosome and lysosome where the cytotoxic drug is released either by cleavage of the linker or degradation of the antibody. Finally, the released payload affects the intracellular target leading to apoptosis and cell death (Hao Chen, Molecule, 2017, 22(8): 1281).

The cytotoxic moiety or payload is the most important element of an ADC. The payloads currently used for ADCs fall into the following four categories: tubulin inhibitor, DNA damaging agent topoisomerase I inhibitor, and RNA polymerase II inhibitor (Hao Chen, Molecule, 2017, 22(8): 1281).

Microtubules, a dynamic assembly of α- and β-tubulin heterodimers are involved in many cellular processes such as cell structure maintenance, cell division, and intracellular transport. Disruption of microtubules induces cell cycle arrest in the G2/M phase. Microtubule/tubulin inhibitors can be classified into two major categories according to their mechanisms of action: agents promoting tubulin polymerization and stabilizing microtubule structures (e.g., paclitaxel, epothilones, discodermolide and taccalonolides), and agents inhibiting tubulin polymerization and destabilizing microtubule structures (such as maytansinoids, auristatins, vinblastine and vincristine) (Hao Chen, Molecule, 2017, 22(8): 1281).

Auristatins have been shown to interfere with microtubule dynamics, GTP hydrolysis and nuclear and cellular division (Woyke et al (2001) Antimicrob. Agents and Chemother. 45(12): 3580-3584) and have anti-cancer (US5663149) and anti-fungal activity (Pettit et al., (1998)Antimicrob. Agents and Chemother. 42 : 2961-2965. The auristatin drug moiety may be attached to the antibody via a linker, through the N (amino) terminus or the C (terminus) of the peptidic drug moiety.

The monomethyl auristatin E (MMAE) is a potent anti-cancer microtubule-targeting agent (MTA) used as a payload in three approved MMAE-containing antibody drug conjugates (ADCs) and multiple ADCs in clinical development to treat different types of cancers. For Example, Tivdak^{™} (tisotumab vedotin) disclosed in WO2011157741A2, is a first-in-class antibody-drug conjugate (ADC) indicated for the treatment of recurrent or metastatic cervical cancer in adult patients whose disease has progressed during or after chemotherapy.

Monopolar Spindle 1 (MPS1) kinase, also known as Tyrosine and Serine/Threonine kinase (TTK) plays a major role in the regulation of mitosis and controls the activity of the spindle assembly checkpoint (SAC). When MPS1 is active, this checkpoint maintains cells in metaphase until chromosome are properly aligned. If MPS1 activity is inhibited, cells undergo mitosis (entering in anaphase) without a correct chromosome alignment. So, the acceleration of mitosis is leading to an increase of aneuploidy and eventually cell death (mitotic catastrophe).

Substituted pyrazolo-quinazoline compounds as monopolar Spindle 1 (MPS1) kinase inhibitors are disclosed in patent application WO2009156315A1. Combinational pharmaceutical treatments of cancer have been adopted by physicians and are typically endowed with several advantages in the treatment of cancer. Examples of such combinations are combination of chemotherapy drugs, combination of chemotherapy plus gene therapy, and chemotherapy plus immunotherapy. However, there are several conditions that have to be met in order to enable the combination therapy to succeed.

A combination of an MPS1 inhibitor and a taxane compound useful for the treatment of cancer is disclosed in WO2019002542A1 and in particular the combination of N-(2,6-diethylphenyl)-8-({4-[4-(dimethylamino)piperidin-1-yl]-2-methoxyphenyl}-amino)-1-methyl-4,5-dihydro-1*H*- pyrazolo[4,3-h]quinazoline-3-carboxamide or one of its salts with a pharmaceutically acceptable acid or base and a taxane compound such as paclitaxel and docetaxel.

However, the severe toxicity associated with the taxane compounds such as the peripheral neuropathy frequently, along with a development of the resistance to taxanes, limits their use in the clinic.

Therefore, there is a long existing need for development of additional safe proven combinational therapies to treat cancer.

### SUMMARY OF THE INVENTION

The present invention provides a combination of two separate types of anticancer drugs to achieve a synergistic effect. Specific combinations of a MPS1 inhibitor, more particularly the compound of formula (I) or one of its salts with a pharmaceutically acceptable acid or base, with an ADC have now been found to control various types of cancer efficiently.

The inventors found out hence a combination of compounds inducing tumor growth delay in the mouse animal model and showing a synergistic effect in inducing the tumor regression.

In a first aspect, the present invention refers to a combination comprising:
(i) N-(2,6-diethylphenyl)-8-({4-[4-(dimethylamino)piperidin-1-yl]-2-methoxyphenyl}-amino)-1-methyl-4,5-dihydro-1H-pyrazolo[4,3-h]quinazoline-3-carboxamide of formula (I) or one of its salts with a pharmaceutically acceptable acid or base 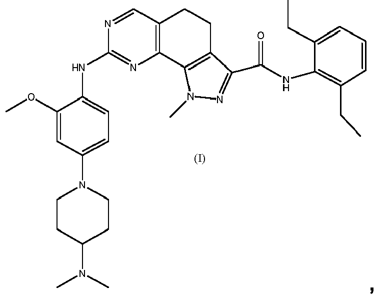 and
(ii) an antibody drug conjugate wherein the payload is a tubulin inhibitor agent,

In one embodiment, the present invention refers to a combination comprising a compound of formula (I) or one of its salts with a pharmaceutically acceptable acid or base, and, tisotumab vedotin as preferred compound (ii).

Advantageously and surprisingly, the administration of the above combination of the invention provides a very efficient control of tumor growth. In particular, such combination treatment provides a synergistic anti-cancer effect, inducing a strong tumor growth delay when compared to vehicle and single agent treated arms. Furthermore, the combination of the present invention provides a good combination benefit with long term tumor regressions.

In a second aspect, the invention refers to a pharmaceutical composition comprising the combination of the present invention, in admixture with one or more pharmaceutically acceptable carrier or excipients.

In a third aspect, the invention refers to a combination of the present invention or a pharmaceutical composition comprising the combination of the present invention for use as a medicament.

In a fourth aspect, the invention refers to a combination of the present invention or a pharmaceutical composition comprising the combination of the present invention for simultaneous, sequential, or separate use.

In a fifth aspect, the invention refers to a combination of the present invention or a pharmaceutical composition comprising the combination of the present invention for use in the treatment of cancer.

All the medical uses, the compositions and combinations and medical treatments of the invention are for the simultaneous, sequential, or separate administration.

In a further aspect, the invention refers to the combination of the present invention and the pharmaceutical composition comprising the combination of the present invention, for use in the treatment of colorectal cancer.

### BRIEF DESCRIPTION OF THE DRAWINGS

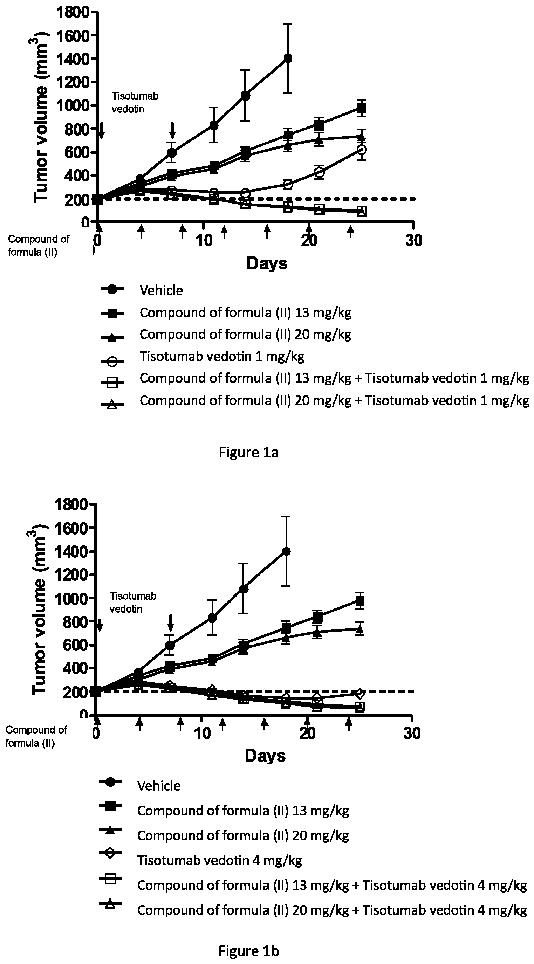

Figure 1a and Figure 1b illustrate the mean tumor volume growth curves of LS411N CRC tumor bearing mice treated with compound of formula (II) as single agent at 13 mg/kg or 20 mg/kg; Tisotumab vedotin single agent at 1 mg/kg or 4 mg/kg; and the combinations of the different doses of compound of formula (II) with Tisotumab vedotin at 1 mg/kg (a) or Tisotumab vedotin at 4 mg/kg (b).

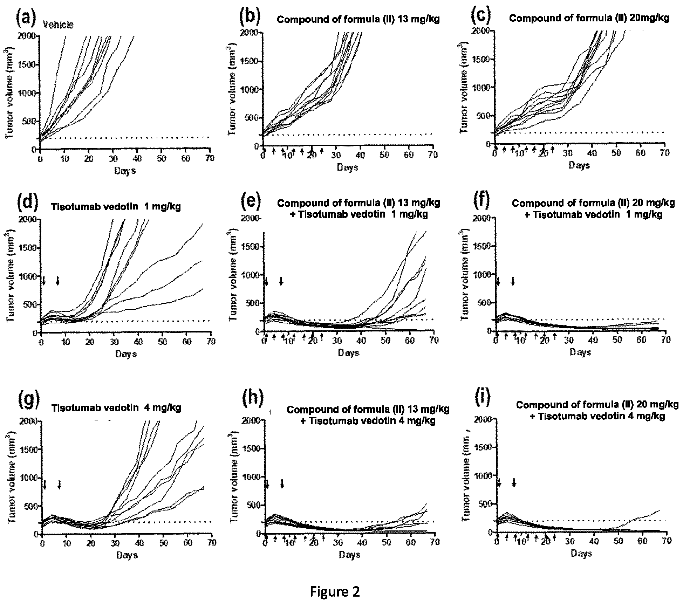

Figure 2 illustrates the individual tumor growth curves of LS411N CRC tumor bearing mice treated with: vehicle (a); compound of formula (II) 13 mg/kg (b); compound of formula (II) 20 mg/kg (c); Tisotumab vedotin 1 mg/kg (d); combination compound of formula (II) 13 mg/kg with Tisotumab vedotin 1 mg/kg (e); combination of compound of formula (II) 20 mg/kg with Tisotumab vedotin 1 mg/kg (f); Tisotumab vedotin 4 mg/kg (g); combination of compound formula (II) 13 mg/kg with Tisotumab vedotin 4 mg/kg (h); combination of compound formula (II) 20 mg/kg with Tisotumab vedotin 4 mg/kg (i). Study cut-off at day 67.

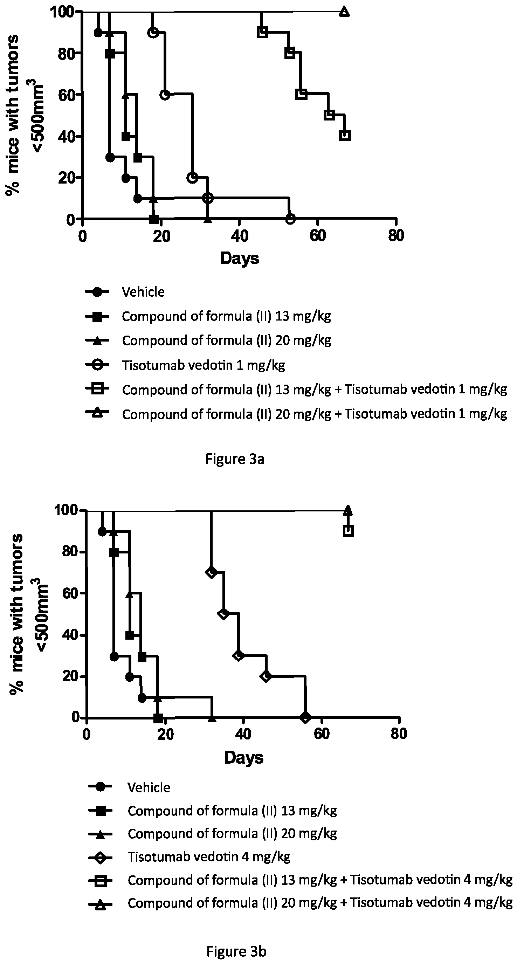

Figure 3a and Figure 3b illustrate the combination benefit of Compound of formula (II) at 13 mg/kg and 20 mg/kg with: Tisotumab vedotin at 1 mg/kg (a) or Tisotumab vedotin at 4 mg/kg (b), when compared to the vehicle and single agent treatment arms, in LS411N xenograft model of CRC. Kaplan-Meier curves with the percentage of mice with tumors <500 mm3 are shown.

### DETAILED DESCRIPTION OF THE INVENTION

The terms "monopolar spindle 1 kinase" or "MPS1" or "TTK protein kinase", are used herein interchangeably.

The terms "antibody drug conjugate" or "ADC" are used herein interchangeably and refer to a class of complex molecules composed of an antibody linked to a biologically active cytotoxic payload or drug.

The terms "monoclonal antibody" or "mAb" or "moAb" are used herein interchangeably and refer to a preparation of antibody molecules of single molecular composition.

The terms "tissue factor", "TF", "tissue factor antigen", "TF antigen" are used interchangeably herein, and, unless specified otherwise, include any variants, isoforms and species homologs of human tissue factor which are naturally expressed by cells or are expressed on cells transfected with the tissue factor gene. The terms "anti-tubulin agent" or "microtubule inhibitor agent" or tubulin inhibitor agent" are used herein interchangeably and refer to an agent that inhibits mitosis, or cell division.

The terms 'co-administration' or 'combined administration' or the like as utilized herein are meant to encompass administration of the selected combination partner to a single subject in need thereof (e.g. a patient), and are intended to include treatment regimens in which the agents are not necessarily administered by the same route of administration or at the same time.

The term 'fixed dose combination' means that the active ingredients, e.g. a compound of formula (I) and one or more combination partners, are both administered to a patient simultaneously in the form of a single entity or dosage.

The term 'non-fixed dose combination' means that the active ingredients, e.g. a compound of the present invention and one or more combination partners, are both administered to a patient as separate entities either simultaneously or sequentially, with no specific time limits, wherein such administration provides therapeutically effective levels of the two compounds in the body of the patient. The latter also applies to cocktail therapy, e.g. the administration of three or more active ingredients.

As used herein, the term "cancer" refers to all types of cancer, neoplasm or malignant tumors found in mammals. As used herein, the term "solid cancer" refers to a mass-type cancer formed in an organ, unlike blood cancer. The term solid cancer includes lymphomas, melanomas, neuroendocrine tumors, carcinomas, carcinosarcoma and Ewing sarcoma and sarcomas. "Sarcomas" are cancers of the connective tissue, cartilage, bone, muscle, and so on. "Carcinomas" are cancers of epithelial (lining) cells. "Adenocarcinoma" refers to carcinoma derived from cells of glandular origin. "Neuroendocrine" tumor is derived from cells that release hormones into the blood. The terms "cancer" and "tumor" are used interchangeably throughout the subject specification. Exemplary cancers that may be treated with a compound, pharmaceutical composition, or method provided herein include lymphoma, sarcoma, bladder cancer, bone cancer, brain tumor, cervical cancer, colon cancer, esophageal cancer, gastric cancer, head and neck cancer, kidney cancer, thyroid cancer, prostate cancer, breast cancer (e.g. triple negative, ER positive, ER negative, chemotherapy resistant, herceptin resistant, HER2 positive, doxorubicin resistant, tamoxifen resistant, ductal carcinoma including palbociclib resistant ductal carcinoma, lobular carcinoma, primary, metastatic), ovarian cancer, pancreatic cancer, lung cancer (e.g. non-small cell lung carcinoma, squamous cell lung carcinoma, adenocarcinoma, large cell lung carcinoma, small cell lung carcinoma, carcinoid, sarcoma), glioblastoma multiforme, glioma, melanoma, prostate cancer, castration-resistant prostate cancer, breast cancer, triple negative breast cancer, glioblastoma, ovarian cancer, lung cancer, squamous cell carcinoma (e.g., head, neck, or esophagus), colorectal cancer, lymphoma, or B cell lymphoma. Additional examples include, cancer of the thyroid, endocrine system, brain, breast, cervix, colon, head & neck, esophagus, liver, kidney, lung, non-small cell lung, melanoma, mesothelioma, ovary, sarcoma, stomach, uterus or medulloblastoma, Hodgkin's disease, non-Hodgkin's lymphoma, neuroblastoma, glioma, glioblastoma multiforme, ovarian cancer, rhabdomyosarcoma, primary thrombocytosis, primary macroglobulinemia, primary brain tumors, cancer, malignant pancreatic insulinoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, endometrial cancer, adrenal cortical cancer, neoplasms of the endocrine or exocrine pancreas, medullary thyroid cancer, medullary thyroid carcinoma, melanoma, colorectal cancer, papillary thyroid cancer, Paget's disease of the nipple, phyllodes tumors, lobular carcinoma, ductal carcinoma, cancer of the pancreatic stellate cells, cancer of the hepatic stellate cells, or prostate cancer.

The term jointly therapeutically effective' means that the therapeutic agents may be given separately (in a chronologically staggered manner, especially a sequence-specific manner) in such time intervals that they prefer, in the warm-blooded animal, especially human, to be treated, still show a (preferably synergistic) interaction (joint therapeutic effect). Whether this is the case can, inter alia, be determined by following the blood levels, showing that both compounds are present in the blood of the human to be treated at least during certain time intervals.

'Synergistically effective' or 'synergy' means that the therapeutic effect observed following administration of two or more agents/compounds is greater than the sum of the therapeutic effects observed following the administration of each single agent. The terms "treating" or "treatment of" a condition or patient refers to taking steps to obtain beneficial or desired results, including clinical results. Beneficial or desired clinical results include, but are not limited to, or ameliorating abrogating, substantially inhibiting, slowing or reversing the progression of a disease, condition or disorder, substantially ameliorating or alleviating clinical or esthetical symptoms of a condition, substantially preventing the appearance of clinical or esthetical symptoms of a disease, condition, or disorder, and protecting from harmful or annoying symptoms. Treating further refers to accomplishing one or more of the following: (a) reducing the severity of the disorder; (b) limiting development of symptoms characteristic of the disorder(s) being treated; (c) limiting worsening of symptoms characteristic of the disorder(s) being treated; (d) limiting recurrence of the disorder(s) in patients that have previously had the disorder(s); and/or (e) limiting recurrence of symptoms in patients that were previously asymptomatic for the disorder(s). The term "treating" with respect to cancer should be understood to e.g. encompass treatment resulting in a decrease in tumor size; a decrease in rate of tumor growth; stasis of tumor size; a decrease in the number of metastasis; a decrease in the number of additional metastasis; a decrease in invasiveness of the cancer; a decrease in the rate of progression of the tumor from one stage to the next; inhibition of tumor growth in a tissue of a mammal having a malignant cancer; control of establishment of metastases; inhibition of tumor metastases formation; regression of established tumors as well as decrease in the angiogenesis induced by the cancer, inhibition of growth and proliferation of cancer cells and so forth. The term "treating cancer" as used herein should also be understood to encompass prophylaxis such as prevention as cancer reoccurs after previous treatment (including surgical removal) and prevention of cancer in an individual prone (genetically, due to life style, chronic inflammation and so forth) to develop cancer. As used herein, "prevention of cancer" is thus to be understood to include prevention of metastases, for example after surgical procedures or after chemotherapy.

As used herein, a subject is 'in need of a treatment if such subject would benefit biologically, medically or in quality of life from such treatment.

As used herein, "safe and effective amount" means an amount of the compound sufficient to treat the patient's condition but low enough to avoid serious side effects and it can nevertheless be routinely determined by the skilled artisan.

The present invention refers to a combination comprising:
(i) N-(2,6-diethylphenyl)-8-({4-[4-(dimethylamino)piperidin-1-yl]-2-methoxyphenyl}-amino)-1-methyl-4,5-dihydro-1H-pyrazolo[4,3-h]quinazoline-3-carboxamide of formula (I) or one of its salts with a pharmaceutically acceptable acid or base 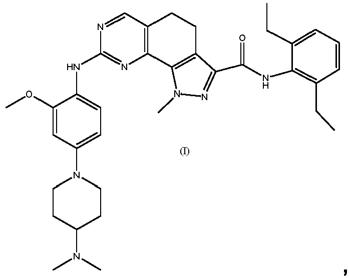 and
(ii) an antibody drug conjugate wherein the payload is a tubulin inhibitor agent Advantageously and surprisingly, the administration of a MPS1 inhibitor, in particular the compound of formula (I) or one of its salts with a pharmaceutically acceptable acid or base, and an antibody drug conjugate wherein the payload is a tubulin inhibitor agent, provides a very efficient control of tumor growth. In fact, as shown in the Experimental part, the administration of said combination to colorectal cancer (CRC) mouse model induces tumor growth inhibition. In particular, such a combination treatment provides a synergistic anti-cancer effect, inducing a strong tumor growth delay when compared to vehicle and single agent treated arms. Furthermore, the combination of the present invention provides a good combination benefit with long term tumor regressions. These results were completely unexpected, it is well-known fact that it is difficult to predict the effect that a combination of drugs would have.

In one embodiment, the invention refers to a combination comprising:
(i) the compound of formula (I) or one of its salts with a pharmaceutically acceptable acid or base, and
(ii) an antibody drug conjugate wherein the payload is a tubulin inhibitor agent, for simultaneous, sequential or separate use.

The combination of the invention is hence for use as a medicament.

In one preferred embodiment the present invention refers to a combination comprising:
(i) the compound of formula (I) or one of its salts with a pharmaceutically acceptable acid or base, and
(ii) an antibody drug conjugate wherein the payload is monomethyl auristatin E, for simultaneous, sequential or separate use.

The combination of the invention comprising as compound ii) an antibody drug conjugate wherein the payload is monomethyl auristatin E is hence for use as a medicament.

In one preferred embodiment, the present invention refers to a combination comprising:
(i) the compound of formula (I) or one of its salts with a pharmaceutically acceptable acid or base, and
(ii) an antibody drug conjugate wherein the antibody is an anti-TF antibody and the payload is a tubulin inhibitor agent,
for simultaneous, sequential or separate use.

The combination of the invention comprising as compound (i) the compound of formula (I) and as compound ii) an antibody drug conjugate wherein the antibody is an anti-TF antibody and the payload is a tubulin inhibitor agent is hence for use as a medicament.

In a more preferred embodiment, the present invention refers to a combination comprising:
(i) the compound of formula (I) or one of its salts with a pharmaceutically acceptable acid or base, and
(ii) an antibody drug conjugate wherein the antibody is an anti-TF antibody and the payload is monomethyl auristatin E,
for simultaneous, sequential or separate use.

The combination of the invention comprising as compound (i) the compound of formula (I) and as compound ii) an antibody drug conjugate wherein the antibody is an anti-TF antibody and the payload is monomethyl auristatin E is hence for use as a medicament.

In an even more preferred embodiment, the present invention refers to a combination comprising:
(i) the compound of formula (I) or one of its salts with a pharmaceutically acceptable acid or base, and
(ii) tisotumab vedotin,
for simultaneous, sequential or separate use.

The combination of the invention comprising as compound ii) tisotumab vedotin is hence for use as a medicament.

In a further preferred embodiment, the present invention refers to a combination comprising:
(i) the compound of formula (I) or one of its salts with a pharmaceutically acceptable acid or base, and
(ii) an antibody drug conjugate wherein the payload is monomethyl auristatin E,selected from the group consisting of sofituzumab vedotin, polatuzumab vedotin, enfortumab vedotin, pinatuzumab vedotin, lifastuzumab vedotin, brentuximab vedotin, blembatumumab vedotin, tisotumab vedotin, yisotumab vedotin, indusatumab vedotin and disitamab vedotin,
for simultaneous, sequential or separate use.

The combination of the invention comprising as compound ii) an antibody drug conjugate wherein the payload is monomethyl auristatin E, selected from the group consisting of sofituzumab vedotin, polatuzumab vedotin, enfortumab vedotin, pinatuzumab vedotin, lifastuzumab vedotin, brentuximab vedotin, blembatumumab vedotin, yisotumab vedotin, indusatumab vedotin and disitamab vedotin is hence for use as a medicament.

Pharmaceutically acceptable salts of the compound of formula (I) include the acid addition salts with inorganic or organic acids, e.g., nitric, hydrochloric, hydrobromic, sulphuric, perchloric, phosphoric, acetic, trifluoroacetic, propionic, glycolic, lactic, oxalic, malonic, malic, maleic, tartaric, citric, benzoic, cinnamic, mandelic, methanesulphonic, benzensulphonic, isethionic and salicylic acid and the like.

In one embodiment the salt of compound of formula (I) is selected from the group consisting of phosphate, methansulphonate (tosylate) and benzensulphonate (besylate).

In a more preferred embodiment, the salt of compound of formula (I) is the phosphate salt, represented by the formula (II)

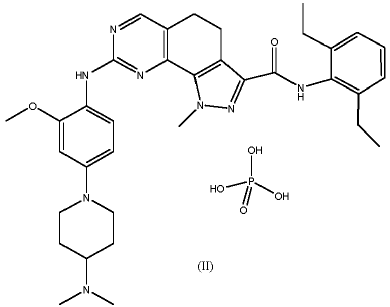

In a more preferred embodiment, the present invention refers to a combination comprising:
(i) the compound of formula (II), and
(ii) tisotumab vedotin.

In a still more preferred embodiment, the present invention refers to a combination comprising:
(i) the compound of formula (II), and
(ii) tisotumab vedotin
for simultaneous, sequential or separate use.

The combination of the invention comprising as compound (i) the compound of Fomula (II) and as compound ii) tisotumab vedotin is hence for use as a medicament.

In one preferred embodiment, the compound of formula (I) or one of its salts with a pharmaceutically acceptable acid or base and the antibody drug conjugate wherein the payload is a tubulin inhibitor agent are administered together.

In an equal preferred embodiment, the compound of formula (I) or one of its salts with a pharmaceutically acceptable acid or base and the antibody drug conjugate wherein the payload is a tubulin inhibitor agent are administered separately.

In a second aspect, the present invention refers to the combination as above described for use in the treatment of cancer.

In one embodiment, the cancer is a cancer which is sensitive to treatment with a tubulin inhibitor.

In one preferred embodiment, the present invention refers to the combination as above described for use in the treatment of cancer such as lymphoma, sarcoma, bladder cancer, bone cancer, brain tumor, cervical cancer, colon cancer, esophageal cancer, gastric cancer, head and neck cancer, kidney cancer, thyroid cancer, prostate cancer, breast cancer (e.g. triple negative, ER positive, ER negative, chemotherapy resistant, herceptin resistant, HER2 positive, doxorubicin resistant, tamoxifen resistant, ductal carcinoma including palbociclib resistant ductal carcinoma, lobular carcinoma, primary, metastatic), ovarian cancer, pancreatic cancer, lung cancer (e.g. non-small cell lung carcinoma, squamous cell lung carcinoma, adenocarcinoma, large cell lung carcinoma, small cell lung carcinoma, carcinoid, sarcoma), glioblastoma multiforme, glioma, melanoma, prostate cancer, castration-resistant prostate cancer, breast cancer, triple negative breast cancer, glioblastoma, ovarian cancer, lung cancer, squamous cell carcinoma (e.g., head, neck, or esophagus), colorectal cancer, lymphoma, or B cell lymphoma.

In a further preferred embodiment, the present invention refers to the combination as above described for use in the treatment of colorectal cancer.

In one aspect, the present invention refers to a method for the treatment of cancer administering to a subject in need thereof, simultaneously, sequentially or separately a combination comprising:
(i) the compound of formula (I) or one of its salts with a pharmaceutically acceptable acid or base, and
(ii) an antibody drug conjugate wherein the payload is a tubulin inhibitor agent. In one embodiment, the invention refers to a method for the treatment of cancer administering to a subject in need thereof, simultaneously, sequentially or separately a combination comprising:
   (i) the compound of formula (I) or one of its salts with a pharmaceutically acceptable acid or base, and
   (ii) an antibody drug conjugate wherein the payload is monomethyl auristatin E. In a more preferred embodiment, the present invention refers to a method for the treatment of cancer administering to a subject in need thereof, simultaneously, sequentially or separately a combination comprising:
      (i) the compound of formula (I) or one of its salts with a pharmaceutically acceptable acid or base, and
      (ii) an antibody drug conjugate wherein the antibody is an anti-TF antibody and the payload is a tubulin inhibitor agent,
for simultaneous, sequential or separate administration.

In a further preferred embodiment, the present invention refers to a method for the treatment of cancer administering to a subject in need thereof, simultaneously, sequentially or separately a combination comprising:
(i) the compound of formula (I) or one of its salts with a pharmaceutically acceptable acid or base, and
(ii) an antibody drug conjugate wherein the antibody is an anti-TF antibody and the payload is monomethyl auristatin E,
for simultaneous, sequential or separate administration.

In an even more preferred embodiment, the invention refers to a method for the treatment of cancer administering to a subject in need thereof, simultaneously, sequentially, or separately a combination comprising:
(i) the compound of formula (II), and
(ii) tisotumab vedotin.

In one embodiment, the invention refers to a method for the treatment of cancer administering to a subject in need thereof, simultaneously, sequentially or separately a combination comprising:
(i) the compound of formula (I) or one of its salts with a pharmaceutically acceptable acid or base, and
(ii) an antibody drug conjugate wherein the payload is monomethyl auristatin E, selected from the group consisting of sofituzumab vedotin, polatuzumab vedotin, enfortumab vedotin, pinatuzumab vedotin, lifastuzumab vedotin, brentuximab vedotin, blembatumumab vedotin, yisotumab vedotin, indusatumab vedotin, disitamab vedotin.

In a preferred embodiment the present invention refers to method for the treatment of cancer such as lymphoma, sarcoma, bladder cancer, bone cancer, brain tumor, cervical cancer, colon cancer, esophageal cancer, gastric cancer, head and neck cancer, kidney cancer, thyroid cancer, prostate cancer, breast cancer (e.g. triple negative, ER positive, ER negative, chemotherapy resistant, herceptin resistant, HER2 positive, doxorubicin resistant, tamoxifen resistant, ductal carcinoma including palbociclib resistant ductal carcinoma, lobular carcinoma, primary, metastatic), ovarian cancer, pancreatic cancer, lung cancer (e.g. non-small cell lung carcinoma, squamous cell lung carcinoma, adenocarcinoma, large cell lung carcinoma, small cell lung carcinoma, carcinoid, sarcoma), glioblastoma multiforme, glioma, melanoma, prostate cancer, castration-resistant prostate cancer, breast cancer, triple negative breast cancer, glioblastoma, ovarian cancer, lung cancer, squamous cell carcinoma (e.g., head, neck, or esophagus), colorectal cancer, lymphoma, or B cell lymphoma.

The compounds of the combination of the invention may moreover be administered in the form of two separate pharmaceutical compositions, each containing one of the active ingredients, or in the form of a single pharmaceutical composition, in which the active ingredients are in admixture.

The useful and preferred dosage regimen of the compound of formula (I) could vary according to the sex, age and weight of the patient, the administration route, the nature of the cancer and of any associated treatments and ranges from 1 mg to 1000 mg of compound of formula (I) per week, more preferably from 20 mg/m² to 50 mg/m², expressed as phosphate salt, per week on Day 1, Day 8 and Day 15 of a 28-days cycle.

The actual dosage levels of the antibody drug conjugate in the combination of the present invention may be varied so as to obtain an amount of the antibody drug conjugate which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

An exemplary, non-limiting range for a therapeutically effective amount of an antibody drug conjugate of the present invention is about 0.1 - 100 mg/kg, such as about 0.1 - 50 mg/kg, for example about 0.1 - 2.0 mg/kg, such as about 0.1- 10 mg/kg, such as about 0.5-5 mg/kg, for instance about 5 mg/kg, such as about 4 mg/kg, or about 3 mg/kg, or about 2 mg/kg, or about 1 mg/kg, or about 0.5 mg/kg, or about 0.3 mg/kg. An exemplary, non-limiting range for a therapeutically effective amount of an antibody drug conjugate of the present invention is about 0.02 - 30 mg/kg, such as about 0.1 - 20 mg/kg, or about 0.5 - 10 mg/kg, or about 0.5 - 5 mg/kg, for example about 1 - 2 mg/kg.

As non-limiting examples, treatment according to the present invention may be provided as a weekly, bi-weekly, three-weekly or monthly dosage of antibody drug conjugate.

In a third aspect the present invention refers to a pharmaceutical composition comprising the combination of the invention in admixture with at least one or more pharmaceutically acceptable carrier or excipients.

In one embodiment, the present invention refers to a pharmaceutical composition comprising the combination of the invention, in admixture with one or more pharmaceutically acceptable carrier or excipients, for example those described in Remington's Pharmaceutical Sciences Handbook, XVII Ed., Mack Pub., N.Y., U.S.A. Administration of the compounds of the invention and their pharmaceutical compositions may be accomplished according to patient's needs, for example, orally, nasally, parenterally and by inhalation.

In one preferred embodiment, the pharmaceutical composition comprising the combination of the invention is administered parenterally, such as epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

In a more preferred embodiment, the pharmaceutical composition comprising the combination of the invention is administered intravenously.

In one embodiment, the compounds of the combination of the invention may moreover be administered in the form of two separate pharmaceutical compositions for intravenous administration, each containing one of the active ingredients, or in the form of a single pharmaceutical composition for intravenous administration, in which the active ingredients are in admixture.

Pharmaceutically acceptable carriers include any and all suitable solvents, dispersion media, coatings, antibacterial and antifungal agents, isotonicity agents, antioxidants and absorption delaying agents, and the like that are physiologically compatible with antibody drug conjugate of the present invention.

Examples of suitable aqueous-and nonaqueous carriers which may be employed in the pharmaceutical compositions comprising the combination of the present invention include water, saline, phosphate buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts.

In one embodiment, the compounds of the combination may be administered in the form of two separate pharmaceutical compositions, wherein the compound of formula (I) is administered orally and the ADC is administered intravenously.

The pharmaceutical composition for oral administration comprising the compound of formula (I) can be a solid oral dosage form such as a tablet, a gelcap, a capsule, a caplet, a granule, a lozenge and bulk powders.

In one preferred embodiment, the pharmaceutical composition comprising the compound of formula (I) is a tablet.

The compound of formula (I) can be combined with various pharmaceutically acceptable carriers, diluents, such as sucrose, mannitol, lactose, starches, and known excipients, including suspending agents, solubilizers, buffering agents, binders, disintegrants, preservatives, colorants, flavorants, lubricants and the like. In a further embodiment, the pharmaceutical composition comprising the combination of the invention is a liquid oral dosage forms such as aqueous and nonaqueous solutions, emulsions, suspensions, syrups. Such liquid dosage forms can also contain suitable known inert diluents such as water and suitable known excipients such as preservatives, wetting agents, sweeteners, flavorants, as well as agents for emulsifying and/or suspending the compounds of the invention.

All preferred groups or embodiments described above for the combination of the invention may be combined among each other and apply as well *mutatis mutandis.* The various aspects of the invention described in this application are illustrated by the following examples which are not meant to limit the invention in any way.

### EXPERIMENTAL PART

### Example 1

LS411N human colorectal cancer cells were maintained in RPMI-1640 cell culture medium supplemented with 10% fetal bovine serum at 37°C in an atmosphere of 5% CO₂ in air. The cells in exponential growth phase were harvested and counted. Balb/c-nude female mice were inoculated subcutaneously at the right flank with 5x106 LS411N cells in 0.2 ml of PBS mixed with Matrigel (1:1) for tumor development. Six days after cell injection, animals having a tumor volume of about 200 mm³ were randomized and allocated to 9 experimental groups (n=10 mice/group) for treatment with vehicle, compound of formula (II) at 13 mg/kg, compound of formula (II) at 20 mg/kg, tisotumab vedotin at 1 mg/kg, tisotumab vedotin at 4 mg/kg and the combinations of the two doses of compound of formula (II) with the two doses of tisotumab vedotin. Compound of formula (II) was administered intravenously every 4 days for 7 times (Days 0, 4, 8, 12, 16, 20 and 24) and tisotumab vedotin was administered intravenously once a week for two weeks (Days 0 and 7). On Day 0, tisotumab vedotin was given first and, after 1h, compound of formula (II) was administered).

During the study, the care and use of animals was conducted in accordance with the regulations of the Association for Assessment an Accreditation of Laboratory Animal Care (AAALAC). Animals were checked for an effects of tumor growth and treatments on normal behavior such as mobility, food and water consumption, body weight gain/loss, eye/hair matting and any other abnormal effects.

Effect of treatment on tumor growth and body weight was evaluated twice times per week. Tumors were measured in two dimensions using a caliper, and the tumor volume was expressed in mm³ using the formula: V=(L x W2)/2, where V is tumor volume, L is tumor length (the longest tumor dimension) and W is tumor width (the longest tumor dimension perpendicular to L).

Mean tumor growth curves are shown in Figure 1a and Figure 1b. From Day 18 onwards, some animals in the vehicle treated groups had to be euthanized due to tumors reaching the ethical endpoint (3000 mm³), therefore tumor growth inhibition (TGI) for each treatment group was calculated on Day 18, when all the animals in the vehicle group were available for the calculation of the mean tumor volume. Mean tumor volumes, SEM and TGI values are shown in Table 1.

**Table 1**

| **Treatment** | **Mean Tumor Volume (mm³) Day 18** | **SEM** | **Comparison vs vehicle *** | **TGI** |
|---|---|---|---|---|
| Vehicle | 1402 | 295 | - | - |
| Compound of formula (II) 13 mg/kg | 751 | 50 | 0.0089 (**) | 46.4% |
| Compound of formula (II) 20 mg/kg | 655 | 56 | 0.0029 (**) | 52.6% |
| Tisotumab vedotin 1 mg/kg | 325 | 37 | <0.0001 (***) | 76.8% |
| Compound of formula (II) 13 mg/kg + tisotumab vedotin 1 mg/kg | 134 | 9 | <0.0001 (***) | 90.5% |
| Compound of formula (II) 20 mg/kg + tisotumab vedotin 1 mg/kg | 124 | 5 | <0.0001(***) | 91.2% |
| Tisotumab vedotin 4 mg/kg | 147 | 12 | <0.0001(***) | 89.5% |
| Compound of formula (II) 13 mg/kg + tisotumab vedotin 4 mg/kg | 115 | 7 | <0.0001 (***) | 91.8% |
| Compound of formula (II) 20 mg/kg + tisotumab vedotin 4 mg/kg | 98 | 4 | <0.0001 (***) | 93.0% |

| | | | | |
|---|---|---|---|---|
| Mann Whitney test* (*p≤0.05, **p≤0.01, ***p≤0.001) | | | | |

Compound of formula (II) and tisotumab vedotin single agents induced a significant TGI. Compound of formula (II) at 13 mg/kg and 20 mg/kg induced a TGI of 46.4% and 52.6%, respectively. Tisotumab vedotin at 1 mg/kg and 4 mg/kg induced a TGI of 76.8% and 89.5%, respectively. All the combinations induced a higher efficacy than the corresponding single agent treatments with TGI values of 90.5%, 91.2%, 91.8%, 93.0% for the combinations Compound of formula (II) 13 mg/kg + tisotumab vedotin 1 mg/kg, Compound of formula (II) 20 mg/kg + tisotumab vedotin 1 mg/kg, Compound of formula (II) 13 mg/kg + tisotumab vedotin 4 mg/kg and Compound of formula (II) 20 mg/kg + tisotumab vedotin 4 mg/kg, respectively. Treatments were well tolerated.

Figure 2 and Figure 3a and Figure 3b show the tumor growth curves of individual animals in each group and the Kaplan-Meier curves representing the time needed for tumors to reach/exceed 500 mm³, respectively. Study cut-off Day 67.

Tisotumab vedotin at 1 mg/kg controlled tumor growth initially, but between Days 14 and 20 all tumors started growing. Tisotumab vedotin at 4 mg/kg controlled tumor growth and induced tumor regressions initially, but after Day 20 tumors started to regrow as well. All the combinations of Compound of formula (II) with tisotumab vedotin provided a good combination benefit with long term tumor regressions. At Day 67, all the combinations had still animals with tumor regressions, specifically 10%, 100%, 70% and 90% tumor regression in the combinations Compound of formula (II) at 13 mg/kg + tisotumab vedotin 1 mg/kg, Compound of formula (II) 20 mg/kg + tisotumab vedotin 1 mg/kg, Compound of formula (II) 13 mg/kg + tisotumab vedotin 4 mg/kg and Compound of formula (II) 20 mg/kg + tisotumab vedotin 4 mg/kg, respectively Table 2.

**Table 2**

| **Treatment** | **Median Time to 500 mm3 (Days)** | **T/C** | **Comparison vs vehicle *** | **Tumor growth delay (Days)** | **Tumor regressions at day 67** |
|---|---|---|---|---|---|
| Vehicle | 7 | | | | 0/10 |
| Compound of formula (II) 13 mg/kg | 11 | 157% | 0.0849 (ns) | 4 | 0/10 |
| Compound of formula (II) 20 mg/kg | 14 | 200% | 0.0323 (*) | 7 | 0/10 |
| Tisotumab vedotin 1 mg/kg | 28 | 400% | <0.0001 (***) | 21 | 0/10 |
| Compound of formula (II) 13 mg/kg + Tisotumab vedotin 1 mg/kg | 65 | 928% | <0.0001 (***) | 58 | 1/10 |
| Compound of formula (II) 20 mg/kg + Tisotumab vedotin 1 mg/kg | Not reached (>67) | >957% | <0.0001 (***) | >60 | 10/10 |
| Tisotumab vedotin 4 mg/kg | 37 | 529% | <0.0001 (***) | 30 | 0/10 |
| Compound of formula (II) 13 mg/kg + Tisotumab vedotin 4 mg/kg | Not reached (>67) | >957% | <0.0001 (***) | >60 | 7/10 |
| Compound of formula (II) 20 mg/kg + Tisotumab vedotin 4 mg/kg | Not reached (>67) | >957% | <0.0001 (***) | >60 | 9/10 |

| | | | | | |
|---|---|---|---|---|---|
| Log-Rank (Mantle Cox) test*: ( *p≤0.05, **p≤0.01, ***p≤0.001) | | | | | |

To better evaluate the combination benefit, tumor growth delay was evaluated as the time needed for tumors to reach/exceed a defined tumor volume (Table 2). The median time for tumors to reach 500 mm³ was 7 days, 11 days, 14 days, 28 days and 37 days for vehicle, Compound of formula (II) 13 mg/kg, Compound of formula (II) 20 mg/kg, tisotumab vedotin 1 mg/kg and tisotumab vedotin 4 mg/kg single agent groups, respectively. All the combinations induced a strong tumor growth delay when compared to vehicle and single agent treated arms. The median time for tumors to reach 500 mm³ in the combination at the lower doses of both agents, Compound of formula (II) 13 mg + tisotumab vedotin 1 mg/kg, was 65 days. For the other combination groups the median time for tumors to reach 500 mm³ had not yet been reached at Day 67. The effect of the combination on tumor growth delay was more than additive for all the combination groups.

## Claims

1. A combination comprising:
(i) N-(2,6-diethyl phenyl)-8-({4-[4-(dimethylamino)piperidin-1-yl]-2-methoxyphenyl}-amino)-1-methyl-4,5-dihydro-1H-pyrazolo[4,3-h]quinazoline-3-carboxamide of formula (I) or one of its salts with a pharmaceutically acceptable acid or base, and 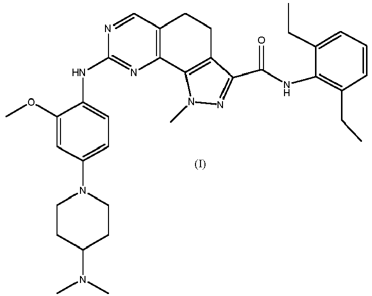
(ii) an antibody drug conjugate wherein the payload is a tubulin inhibitor agent.

2. The combination according to claim 1, comprising
(i) compound of formula (I) or one of its salts with a pharmaceutically acceptable acid or base and
(ii) an antibody drug conjugate wherein the payload is monomethyl auristatin E.

3. The combination according to claim 1 comprising:
(i) a compound of formula (I) or one of its salts with a pharmaceutically acceptable acid or base, and
(ii) an antibody drug conjugate wherein the antibody is an anti-TF antibody and the payload is tubulin inhibitor agent.

4. The combination according to anyone of claims 1 to 3 comprising:
(i) a compound of formula (I) or one of its salts with a pharmaceutically acceptable acid or base, and
(ii) an antibody drug conjugate wherein the antibody is an anti-TF antibody and the payload is monomethyl auristatin E.

5. The combination according to anyone of claims 1 to 4 comprising:
(i) the compound of formula (I) or one of its salts with a pharmaceutically acceptable acid or base, and
(ii) tisotumab vedotin.

6. The combination according to anyone of claims 1 to 5, wherein the salt of compound of formula (I) is the phosphate salt, represented by the formula (II)

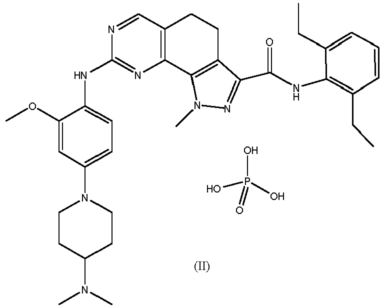

7. The combination according to anyone of claims 1 to 6, comprising:
(i) the compound of formula (II) and
(ii) tisotumab vedotin.

8. The combination according to claims 1 or 2, comprising:
(i) the compound of formula (I) or one of its salts with a pharmaceutically acceptable acid or base and
(ii) an antibody drug conjugate wherein the payload is monomethyl auristatin E, selected from the group consisting of sofituzumab vedotin, polatuzumab vedotin, enfortumab vedotin, pinatuzumab vedotin, lifastuzumab vedotin, brentuximab vedotin, blembatumumab vedotin, tisotumab vedotin, indusatumab vedotin and disitamab vedotin.

9. A pharmaceutical composition comprising the combination according to anyone of claims 1 to 8 in admixture with one or more pharmaceutically acceptable carrier or excipients.

10. A combination according to anyone of claims 1 to 8 for use as a medicament.

11. The combination according to claim 10, for simultaneous, sequential or separate use.

12. The combination for use according to claim 10, wherein the compound of formula (I) or one of its salts with a pharmaceutically acceptable acid or base and the antibody drug conjugate wherein the payload is a tubulin inhibitor agent are administered together.

13. The combination for use according to claim 10, wherein the compound of formula (I) or one of its salts with a pharmaceutically acceptable acid or base and the antibody drug conjugate wherein the payload is a tubulin inhibitor agent are administered separately.

14. The combination for use according to claims 10 or 11, wherein compound of formula (I) or one of its salts with a pharmaceutically acceptable acid or base and the antibody drug conjugate wherein the payload is a tubulin inhibitor agent are administered in the form of two separate pharmaceutical compositions.

15. The combination for use according to claims 10 or 11, wherein compound of formula (I) or one of its salts with a pharmaceutically acceptable acid or base and the antibody drug conjugate wherein the payload is a tubulin inhibitor agent are administered in the form of a single pharmaceutical composition.

16. The combination for use according to anyone of claims 10 to 15, wherein the compound of formula (I) or one of its salts with a pharmaceutically acceptable acid or base and the antibody drug conjugate wherein the payload is a tubulin inhibitor agent are administered intravenously.

17. The combination for use according to anyone of claims 10 to 15 wherein the compound of formula (I) or one of its salts with a pharmaceutically acceptable acid or base is administered orally and the antibody drug conjugate wherein the payload is a tubulin inhibitor agent is administered intravenously.

18. A combination according to anyone of claims 1 to 8 or the pharmaceutical composition according to claim 9 for use in the treatment of cancer.

19. The combination or the composition for use according to claim 18, wherein the dose of the compound of formula (II) is from 20 mg/m² to 50 mg/m² per week.

20. The combination or the composition for use according to claim 18, wherein the dose of the antibody drug conjugate wherein the payload is a tubulin inhibitor agent is from 0.1 - 100 mg/kg per week.

21. The combination or the pharmaceutical composition for use according to claim 18, wherein the cancer is selected from the group consisting of lymphoma, sarcoma, bladder cancer, bone cancer, brain tumor, cervical cancer, colon cancer, esophageal cancer, gastric cancer, head and neck cancer, kidney cancer, thyroid cancer, prostate cancer, breast cancer (e.g. triple negative, ER positive, ER negative, chemotherapy resistant, herceptin resistant, HER2 positive, doxorubicin resistant, tamoxifen resistant, ductal carcinoma including palbociclib resistant ductal carcinoma, lobular carcinoma, primary, metastatic), ovarian cancer, pancreatic cancer, lung cancer (e.g. non-small cell lung carcinoma, squamous cell lung carcinoma, adenocarcinoma, large cell lung carcinoma, small cell lung carcinoma, carcinoid, sarcoma), glioblastoma multiforme, glioma, melanoma, prostate cancer, castration-resistant prostate cancer, breast cancer, triple negative breast cancer, glioblastoma, ovarian cancer, lung cancer, squamous cell carcinoma (e.g., head, neck, or esophagus), colorectal cancer, lymphoma and B cell lymphoma.

22. The combination or the pharmaceutical composition for use according to claim 21 wherein the cancer is colorectal cancer.
